# EUROPEAN PATENT SPECIFICATION

(11) **EP 1 704 889 B1**
(45) Date of publication and mention of the grant of the patent: **17.03.2010**
(21) Application number: 04702807.1
(22) Date of filing: 16.01.2004
(51) Int. Cl.: A61M 25/06

(54) **INDWELLING NEEDLE**
VERWEILNADEL
AIGUILLE A DEMEURE

(43) Date of publication of application: 27.09.2006
(73) Proprietor: KABUSHIKI KAISHA TOP, Tokyo 120-0035 (JP)
(72) Inventor: OSHIMA, Hiroshi, c/o KABUSHIKI KAISHA TOP, Tokyo 120-0035 (JP)
(74) Representative: Henkel, Feiler & Hänzel
(86) International application number: PCT/JP2004/000268
(87) International publication number: WO 2005/068006

(56) References cited:
- EP-A1- 0 545 671
- EP-A2- 0 815 887
- EP-B1- 0 734 739
- JP-A- 10 201 852
- JP-A- 2002 119 589
- US-A- 5 135 502
- US-A- 5 738 660
- US-B1- 6 261 259

## Description

### Technical Field

The present invention relates to an indwelling needle used for injecting a drug solution.

### Background Art

A known indwelling needle includes an inner needle having an edge at its tip, an inner needle hub connected to a bottom of the inner needle, a hollow outer needle into which the inner needle is slidably inserted, an outer needle hub connected to a bottom of the outer needle, and a cylindrical protector surrounding the outer needle across an exposed portion of the edge of the inner needle in a state before puncture where the edge of the inner needle protrudes from a tip of the outer needle (for example, Japanese Patent Laid-Open No. 10-201852).

In the indwelling needle, the outer needle hub and the inner needle hub are slidably inserted into the protector to cause a tip of the inner needle hub to abut against a bottom of the outer needle hub. In the indwelling needle, an engagement mechanism for controlling protrusion of the inner needle hub from a tip of the protector is provided at the tip of the protector, and when the protector is held and retracted axially rearward after puncture, the inner needle hub is retracted together with the protector by the engagement mechanism to withdraw the inner needle from the outer needle.

Since the indwelling needle has the engagement mechanism at the tip of the protector, puncture cannot be performed with the tip of the protector abutted against a puncture site of a patient. Thus, before puncture, a health care worker has to push the inner needle hub to advance the inner needle hub and the outer needle hub axially forward relative to the protector, and protrude the outer needle from the tip of the protector with the inner needle inserted.

With the indwelling needle, after insertion of the indwelling needle into the patient, the health care worker withdraws only the inner needle with the outer needle still pierced into the patient, and then the inner needle hub is retracted axially rearward relative to the protector to place the inner needle into the protector.

However, with such a conventional indwelling needle, there is a possibility that the health care worker accidentally touches the inner needle with his/her finger during the time between exposing the inner needle and the outer needle from the protector and insertion thereof or between withdrawing the inner needle from the patient and placing the inner needle into the protector after the insertion.

US 5135 502 discloses an indwelling needle with protections with a needle surrounded by a catheter covered by telescoping containers which expose the insertion ends of the needle and catheter only at the time of insertion.

### Disclosure of the Invention

The invention has an object to provide an indwelling needle that solves the above described inconvenience and reliably prevents a health care worker from accidentally touching an inner needle with his/her finger.

In order to achieve the object, an indwelling needle according to the invention is an improvement, as described below, of an indwelling needle including: an inner needle having an edge at its tip; an inner needle hub connected to a bottom of the inner needle; a hollow outer needle into which the inner needle is slidably inserted; an outer needle hub connected to a bottom of the outer needle; and a cylindrical protector surrounding the outer needle across an exposed portion of the edge of the inner needle in a state before puncture where the edge of the inner needle protrudes from a tip of the outer needle.

Specifically, in the invention, a sleeve that abuts against the outer needle hub to prevent the outer needle hub from retracting axially rearward relative to the inner needle hub is provided outside the inner needle hub, the protector is axially movably provided outside the sleeve, a first engagement mechanism capable of elastically engaging the protector in an advanced end position axially forward relative to the sleeve and a second engagement mechanism capable of engaging the protector in a retracted end position axially rearward relative to the sleeve are provided between the sleeve and the protector, an extending portion extending farther outward from the protector is provided on the outer needle hub, and an inserting portion into which the protector is axially inserted is formed in the extending portion.

Further, a tip of the protector is placed farther forward from the edge of the inner needle protruding from the tip of the outer needle in the advanced end position of the protector, the tip of the protector is placed in substantially the same position as the extending portion in the retracted end position, a retaining mechanism capable of elastically preventing the protector from being withdrawn axially rearward from the inserting portion is provided between the outer needle hub and the protector, further, the inner needle hub is retractable axially rearward relative to the sleeve, a stopper mechanism for preventing further retraction of the inner needle hub relative to the sleeve when the inner needle hub is retracted relative to the sleeve to a predetermined position where the edge of the inner needle is inserted into the protector placed in the retracted end position is provided between the inner needle hub and the sleeve, and when the inner needle hub is further retracted axially rearward from the predetermined position with the extending portion pressed, the protector is withdrawn axially rearward from the inserting portion against an engagement force by the retaining mechanism, by a pulling force axially rearward acting on the protector via the stopper mechanism, the sleeve, and the second engagement mechanism.

According to the invention, there is no obstacle such as the conventional engagement mechanism at the tip of the protector, thereby allowing the tip of the protector to be abutted against a puncture site of a patient. If the tip of the protector is abutted against the puncture site of the patient with the protector engaged in the advanced end position, and the inner needle hub is pushed axially forward from this state, the outer needle hub is advanced axially forward together with the inner needle hub via the sleeve. On the other hand, a drag force of skin in the puncture site is applied to the protector to release the engagement by the first engagement mechanism and cause the protector to retract relative to the inner needle hub and the outer needle hub. As a result, the inner needle and the outer needle protrude from the tip of the protector and inserted into the patient. In a puncture completion state, the protector reaches the retracted end position, and the extending portion of the outer needle hub is seated around the puncture site. Then, the inner needle hub is retracted axially rearward with the extending portion pressed to withdraw the inner needle, but the retaining mechanism prevents the protector from retracting until the inner needle is retracted to the predetermined position, and the inner needle is pulled back to the predetermined position where the edge of the inner needle is inserted into the protector. When the inner needle hub is retracted to the predetermined position relative to the sleeve, the stopper mechanism prevents further retraction of the inner needle hub relative to the sleeve. If the inner needle hub is further retracted from the predetermined position, the protector is withdrawn axially rearward from the inserting portion of the outer needle hub by the pulling force axially rearward acting on the protector via the stopper mechanism, the sleeve, and the second engagement mechanism. Then, the inner needle is placed in the protector.

In this way, puncture can be performed with the protector always surrounding the inner needle and the outer needle and without operation of exposing the inner needle and the outer needle from the protector when the indwelling needle is inserted. This reliably prevents a health care worker from accidentally touching the inner needle or the outer needle with his /her finger before insertion of the indwelling needle.

When the inner needle is withdrawn after the puncture, the inner needle is housed in the protector as it is. This reliably prevents a health care worker from accidentally touching the inner needle with his/her finger after the insertion of the indwelling needle.

The sleeve is movable axially forward from an initial position where the outer needle hub is prevented from retracting axially rearward relative to the inner needle hub to a position where the sleeve is controlled by the stopper mechanism, and thus when the inner needle hub is held and lifted, the sleeve falls under its own weight to the position where the sleeve is controlled by the stopper mechanism to extend the indwelling needle, which becomes difficult to operate. In this case, providing, between the sleeve and the inner needle hub, a third engagement mechanism capable of elastically engaging the sleeve in the initial position allows the sleeve to be kept in the initial position even if the inner needle hub is lifted, thus advantageously preventing extension of the indwelling needle.

A first engagement hole at the tip and a second engagement hole at the bottom radially passing through are formed in the sleeve, a first engagement piece is formed at the bottom of the protector, which engages in the first engagement hole when the protector is placed in the advanced end position relative to the sleeve, and engages in the second engagement hole when the protector is placed in the retracted end position relative to the sleeve, and the first engagement piece and the first engagement hole constitute the first engagement mechanism, and further, a second engagement piece is formed at the tip of the inner needle hub, which engages in the first engagement hole when the sleeve is placed in the initial position, and engages in the second engagement hole when the inner needle hub is retracted to the predetermined position, the second engagement piece and the first engagement hole constitute the third engagement mechanism, and the second engagement piece and the second engagement hole constitute the stopper mechanism. This provides a simple structure of the first to the third engagement mechanisms and the stopper mechanism, advantageously allowing cost reduction.

In this case, if an axial groove connecting the first engagement hole and the second engagement hole is formed in an outer peripherally of the sleeve so as to guide the first engagement piece when the protector moves axially rearward from the advanced end position to the retracted end position relative to the sleeve, the first engagement piece engages in the groove in the outer periphery when the protector moves from the advanced end position to the retracted end position relative to the sleeve 7 to prevent phase shift of the protector relative to the sleeve and ensure engagement of the first engagement piece in the second engagement hole in the retracted end position.

If an axial groove connecting the first engagement hole and the second engagement hole are formed in an inner peripherally of the sleeve so as to guide the second engagement piece when the inner needle hub moves axially rearward from the initial position to the predetermined position relative to the sleeve, the second engagement piece engages in the groove in the inner periphery when the inner needle hub is retracted from the initial position to the predetermined position relative to the sleeve to prevent phase shift of the inner needle hub relative to the sleeve and ensure engagement of the second engagement piece in the second engagement hole in the predetermined position.

If a flange is formed at the bottom of the inner needle hub, and a substantially cylindrical grip extending axially forward and having an inner diameter larger than an outer diameter of the protector is formed on an outer periphery of the flange, holding the grip facilitates axial push-pull operation of the inner needle hub. In puncture, the protector is retracted axially rearward relative to the inner needle hub. Since the inner diameter of the grip is larger than the outer diameter of the protector, the retraction of the protector is not interrupted by the grip.

### Brief Description of the Drawings

FIG. 1 is a sectional view of an indwelling needle according to an embodiment of the invention;
FIG. 2 is a perspective view of the indwelling needle according to the embodiment in an exploded state;
FIG. 3A is a sectional view of the indwelling needle according to the embodiment in a puncture completion state;
FIG. 3B is a sectional view of a state where an inner needle of the indwelling needle according to the embodiment is pulled back to a position where an edge thereof is inserted into a protector; and
FIG. 3C is a sectional view of a state where retaining of an outer needle hub relative to the protector of the indwelling needle according to the embodiment is released.

### Best Mode for Carrying Out the Invention

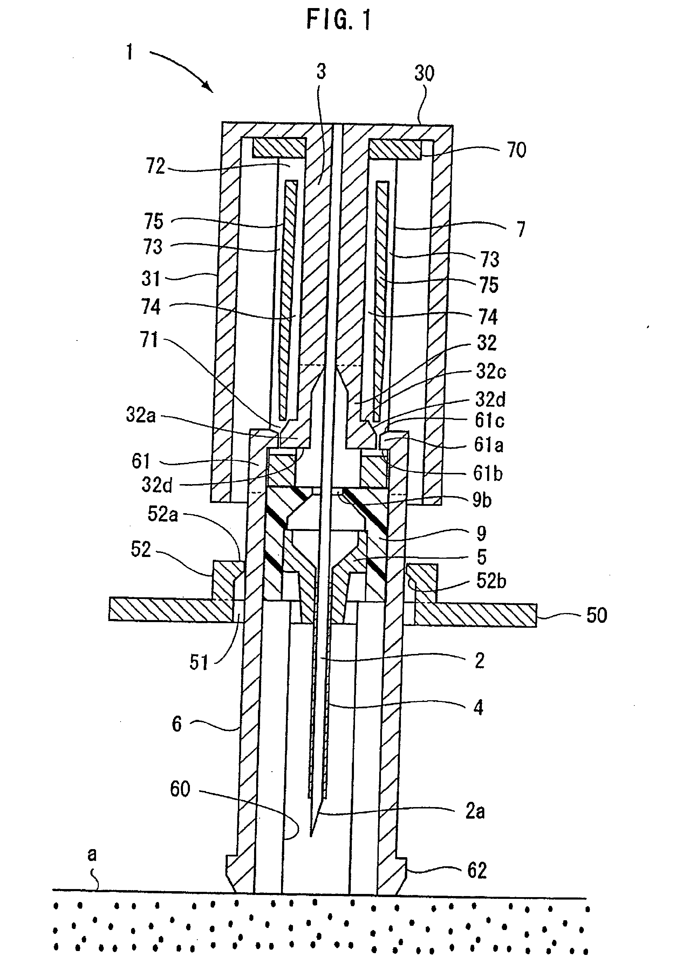

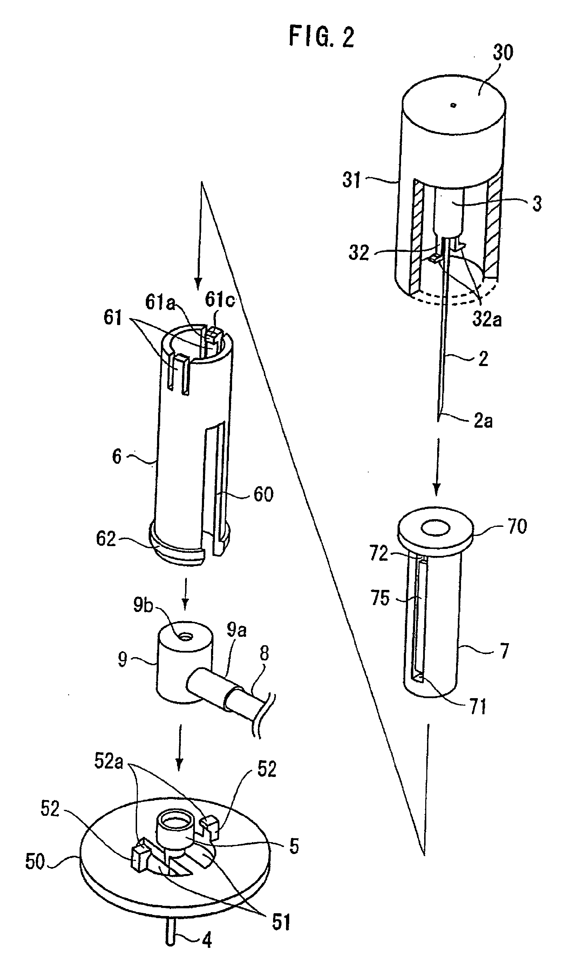

As shown in FIGS. 1 and 2, an indwelling needle 1 according to an embodiment of the invention includes an inner needle 2 having an edge 2a at its tip, an inner needle hub 3 connected to a bottom of the inner needle 2, a hollow outer needle 4 into which the inner needle 2 is slidably inserted, a substantially cylindrical outer needle hub 5 connected to a bottom of the outer needle 4, and a cylindrical protector 6 surrounding the outer needle 4 across an exposed portion of the edge 2a of the inner needle 2 in a state before puncture where the edge 2a of the inner needle 2 protrudes from a tip of the outer needle 4.

A sleeve 7 is provided outside the inner needle hub 3. The sleeve 7 has, at its bottom, a flange 70 abutting against a flange 30 formed at a bottom of the inner needle hub 3. A tip of the sleeve 7 is abutted against the outer needle hub 5 to prevent the outer needle hub 5 from retracting axially rearward relative to the inner needle hub 3. A cover 9 having a joint 9a to which a tube 8 for injecting a drug solution is fitted to a bottom of the outer needle hub 5, and the sleeve 7 abuts against the outer needle hub 5 via the cover 9. A hole 9b into which the inner needle 2 can be inserted is formed in a bottom surface of the cover 9. A substantially cylindrical grip 31 extending axially forward is formed on an outer periphery of the flange 30. An inner diameter of the grip 31 is larger than an outer diameter of the protector 6.

A first engagement hole 71 at the tip and a second engagement hole 72 at the bottom radially passing through are formed in both sides of a periphery of the sleeve 7. Axial grooves 73, 74 connecting the first engagement hole 71 and the second engagement hole 72 are formed in an outer periphery and an inner periphery of the sleeve 7 so as to leave a thin plate 75 between the groove 73 in the outer periphery and the groove 74 in the inner periphery.

The protector 6 is axially movably provided outside the sleeve 7. A pair of wide slits 60 extending axially rearward from the tip are formed in both sides of a periphery of the protector 6. The joint 9a of the cover 9 protrudes outward from the protector 6 through one of the slits 60.

A disk-like extending portion 50 extending farther outward from the protector 6 is formed on the tip of the outer needle hub 5. The extending portion 50 has a pair of inserting portions 51 constituted by substantially semicircular holes. A half cylindrical portion of the protector 6 divided by the slit 60 is inserted into each inserting portion 51.

A pair of first engagement pieces 61 placed on both sides of the periphery are formed at the bottom of the protector 6. Each first engagement piece 61 can elastically bend radially outward. A protrusion 61a protruding radially inward is formed at an end axially rearward (upward in FIG. 1) of each first engagement piece 61. A surface axially forward of the protrusion 61a (the lower surface in FIG. 1) is formed in a flat surface 61b perpendicular to the axial direction. An inclined surface 61c axially inclined is formed in a surface axially rearward of the protrusion 61a (the upper surface in FIG. 1).

The first engagement piece 61 engages, at the protrusion 61a, in the first engagement hole 71 at the tip of the sleeve 7, when the protector 6 is placed in an advanced end position axially forward relative to the sleeve 7. When the protector 6 is pushed axially rearward from the advanced end position, the inclined surface 61c of the protrusion 61a abuts against a tip of the plate 75, the first engagement piece 61 bends outward by a component of force acting via the inclined surface 61c, and the protrusion 61a is disengaged from the first engagement hole 71 to release engagement in the advanced end position. Thus, the first engagement hole 71 and the first engagement piece 61 constitute a first engagement mechanism capable of elastically engaging the protector 6 in the advanced end position. When the protector 6 is placed in the advanced end position, the tip of the protector 6 is placed farther forward from the edge 2a of the inner needle 2 protruding from the tip of the outer needle 4.

When the protector 6 is placed in a retracted end position axially rearward relative to the sleeve 7, the first engagement piece 61 engages, at the protrusion 61a, in the second engagement hole 72 at the bottom of the sleeve 7.

In this state, the flat surface 61b of the protrusion 61a abuts against a bottom of the plate 73, so that the protector 6 cannot move axially forward relative to the sleeve 7. Thus, the second engagement hole 72 and the first engagement piece 61 constitute a second engagement mechanism capable of engaging the protector 6 in the retracted end position. When the protector 6 is placed in the retracted end position, the tip of the protector 6 is placed in substantially the same position as the extending portion 5a.

When the protector 6 moves from the advanced end position to the retracted end position relative to the sleeve 7, the first engagement piece 61 engages in the groove 73 in the outer periphery of the sleeve 7 and is guided to prevent phase shift (rotation) of the protector 6 relative to the sleeve 7 and ensure engagement of the first engagement piece 61 in the second engagement hole 72 in the retracted end position.

The outer needle hub 5 has a pair of third engagement pieces 52 extending axially rearward formed on peripheral edges radially outward from the pair of inserting portions 51. Each third engagement pieces 52 can elastically bend radially outward. A protrusion 52a protruding radially inward is formed at an end axially rearward (upward in FIG. 1) of each third engagement piece 52. An inclined surface 52b axially inclined is formed in a surface axially forward of the protrusion 52a (the lower surface in FIG. 1). A larger diameter portion 62 is formed at the tip of the protector 6 so as to protrude radially outward.

The larger diameter portion 62 engages the protrusion 52a of the third engagement piece 52 when the protector 6 is placed in the retracted end position. If the protector 6 is pulled axially rearward from this state, the third engagement piece 52 bends outward by a component of force acting via the inclined surface 52b of the protrusion 52a to release retaining of the protector 6. Thus, the third engagement piece 52 and the larger diameter portion 62 constitute a retaining mechanism capable of elastically preventing the protector 6 from being withdrawn axially rearward from the inserting portion 51.

The inner needle hub 3 is retractable axially rearward relative to the sleeve 7. A pair of second engagement pieces 32 placed on both sides of the periphery are formed at the tip of the inner needle hub 3. Each second engagement piece 32 can elastically bend radially inward. A protrusion 32a protruding radially outward is formed at an end axially forward (downward in FIG. 1) of each second engagement piece 32. A surface axially forward of the protrusion 32a (the lower surface in FIG. 1) is formed in a flat surface 32b perpendicular to the axial direction. A surface axially rearward of the protrusion 32 (the upper surface in FIG. 1) is constituted by a flat surface 32c placed radially inward and perpendicular to the axial direction and an inclined surface 32d placed radially outward and axially inclined.

The second engagement piece 32 engages, at the protrusion 32a, in the first engagement hole 71 at the tip of the sleeve 7, when the flange 70 at the bottom of the sleeve 7 abuts against the flange 30 at the bottom of the inner needle hub 3, and the sleeve 7 is placed in an initial position where the outer needle hub 5 is prevented from retracting axially rearward relative to the inner needle hub 3. When the sleeve 7 is pulled axially forward relative to the inner needle hub 3 from this state, the tip of the plate 7d abuts against the inclined surface 32d of the protrusion 32a, the second engagement piece 32 bends inward by a component of force acting via the inclined surface 32d to release engagement in the initial position. Thus, the first engagement hole 71 and the second engagement piece 32 constitute a third engagement mechanism capable of elastically engaging the sleeve 7 in the initial position relative to the inner needle hub 3.

When the inner needle hub 3 is retracted relative to the sleeve 7 to a predetermined position where the edge 2a of the inner needle 2 is inserted into the protector 6 placed in the retracted end poison, the second engagement piece 32 engages, at the protrusion 32a, in the second engagement hole 72 at the bottom of the sleeve 7.

In this state, the flat surface 32c of the protrusion 32a abuts against a surface on the bottom side of the second engagement hole 72 (the upper surface in FIG. 1), so that the inner needle hub 3 cannot move axially rearward relative to the sleeve 7. Thus, the second engagement hole 72 and the second engagement piece 32 constitute a stopper mechanism for preventing the inner needle hub 3 from retracting relative to the sleeve 7 beyond the predetermined position.

When the inner needle hub 3 is retracted from the initial position to the predetermined position relative to the sleeve 7, the second engagement piece 32 engages in the groove 74 in the inner periphery of the sleeve 7 and is guided to prevent phase shift (rotation) of the inner needle hub 3 relative to the sleeve and ensure engagement of the second engagement piece 32 in the second engagement hole 72 in the predetermined position.

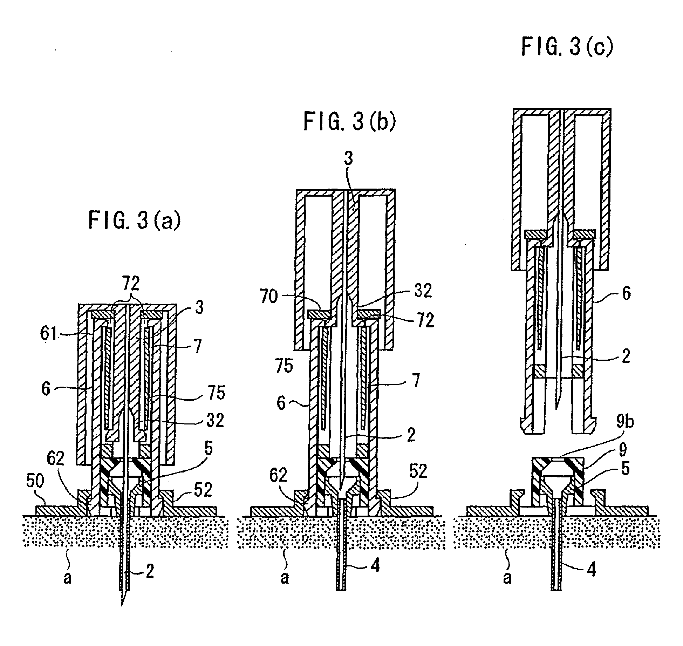

Next, a use of the indwelling needle 1 according to the embodiment will be described in detail with reference to FIGS. 1 and 3.

When the indwelling needle 1 according to the embodiment is used, as shown in FIG. 1, the tip of the protector 6 is first abutted against a puncture site of a patient with the protector 6 engaged in the advanced end position. If the inner needle hub 3 is pushed axially forward from this state, the outer needle hub 5 is advanced axially forward together with the inner needle hub 3 via the sleeve 7 and the cover 9.

On the other hand, a drag force of skin *a* in the puncture site is applied to the protector 6, so that the tip of the plate 75 abuts against the inclined surface 61c in the protrusion 61a of the first engagement piece 61. Then, the first engagement piece 61 bends outward by the component of force acting via the inclined surface 61c to release the engagement by the first engagement mechanism. This causes the protector 6 to retract relative to the inner needle hub 3 and the outer needle hub 5. As a result, the inner needle 2 and the outer needle 4 protrude from the tip of the protector 6 to be inserted into the patient.

Since the inner diameter of the grip 31 is larger than the outer diameter of the protector 6, the retraction of the protector 6 is not interrupted by the grip 31. In puncture, the protector 6 presses the skin *a* around the puncture site of the patient until the engagement by the first engagement mechanism is released in the advanced end position of the protector 6 , thereby relieving pain in the patient in puncture.

In a puncture completion state, as shown in FIG. 3A, the protector 6 is retracted to the retracted end position relative to the sleeve 7, the first engagement piece 61 engages in the second engagement hole 72 at the bottom of the sleeve 7, and the protector 6 is engaged in the rear end position by the second engagement mechanism. At this time, the extending portion 50 of the outer needle hub 5 is seated around the puncture site.

Then, the inner needle hub 3 is retracted axially rearward with the extending portion 50 pressed to withdraw the inner needle 2. An engagement force by the retaining mechanism, that is, an engagement force generated by abutment between the larger diameter portion 62 and the inclined surface 52b in the protrusion 52a of the third engagement piece 52 is set stronger than an engagement force by the third engagement mechanism, that is, an engagement force generated by abutment between the inclined surface 32d in the protrusion 32a of the second engagement piece 32 and the tip of the plate 75.

Thus, the protector 6 is not retracted by the engagement force generated by the retaining mechanism until the inner needle hub 3 is retracted to the predetermined position where the edge 2a of the inner needle 2 is inserted into the protector 6.

Since the sleeve 7 is also engaged to the protector 6 by the second engagement mechanism, the sleeve 7 is not retracted. Thus, the inclined surface 32c in the protrusion 32a of the second engagement piece 32 abuts against the plate 73, the second engagement piece 32 bends inward by the component of force acting via the inclined surface 32d to release the engagement by the third engagement mechanism, and as shown in FIG. 3B, the inner needle hub 3 is pulled back to the predetermined position where the edge 2a of the inner needle 2 is inserted into the protector 6.

When the inner needle hub 3 is pulled back to the predetermined position, the second engagement piece 32 engages in the second engagement hole 72 at the bottom of the sleeve 7, and the stopper mechanism prevents further retraction of the inner needle hub 3 relative to the sleeve 7.

Then, if the inner needle hub 3 is further retracted axially rearward, the sleeve 7 is also retracted together with the inner needle hub 3 via the stopper mechanism, and a pulling force is also transmitted to the protector 6 via the second engagement mechanism. The third engagement piece 52 bends outward by the component of force acting on the inclined surface 52b in the protrusion 52a of the third engagement piece 52 via the larger diameter portion 62 of the protector 6 to release retaining by the retaining mechanism. The inner needle 2 is, as shown in FIG. 3C, then withdrawn with the protector 6 surrounding the inner needle 2, and disposed of as it is.

In this state, the bottom of the plate 75 abuts against the flat surface 32b in the protrusion 32a of the second engagement piece 32, so that the sleeve 7 cannot retract axially rearward relative to the inner needle hub 3. Further, the protector 6 also abuts, at its bottom, against the flange 70 of the sleeve 7, and thus cannot retract axially rearward relative to the sleeve 7. This prevents the inner needle 2 from protruding axially forward again from the protector 6.

The hole 9b opens into the bottom surface of the cover 9, and the hole 9b is sealed with a medical tape (not shown) after the inner needle 2 is withdrawn, or a valve member (not shown) is previously provided in the hole 9b to automatically seal the opening of the hole 9b when the inner needle 2 is withdrawn. Then, a drug solution supplied through the tube 8 is injected into the patient via the cover 9, the outer needle hub 5, and the outer needle 4.

According to the indwelling needle 1 of the embodiment, puncture can be performed with the protector 6 always surrounding the inner needle 2 and the outer needle 4, thereby reliably preventing a health care worker from accidentally touching the inner needle or the outer needle with his/her finger before insertion of the indwelling needle 1.

When the inner needle 2 is withdrawn after the puncture, the inner needle 2 is withdrawn from the outer needle 4 and, at the same time, housed in the protector 6 as it is, thereby reliably preventing a health care worker from accidentally touching the inner needle with his/her finger after the insertion of the indwelling needle 1.

In the initial state in FIG. 1, the second engagement piece 32 engages in the first engagement hole 71, and the sleeve 7 is engaged in the initial position relative to the inner needle hub 3 by the third engagement mechanism. Thus, even if the inner needle hub 3 is lifted, the sleeve 7 does not fall under its own weight to extend the indwelling needle 1, thereby improving operability in puncture.

The substantially cylindrical grip 31 extending axially forward is formed on the outer periphery of the flange 30 of the inner needle hub 3, and thus holding the grip 31 facilitates axial push-pull operation of the inner needle hub 3.

## Claims

1. An indwelling needle (1) comprising:
an inner needle (2) having an edge (2a) its tip;
an inner needle hub (3) connected to a bottom of the inner needle;
a hollow outer needle (4) into which the inner needle is slidably inserted;
an outer needle hub (5) connected to a bottom of the outer needle; and
a cylindrical protector (6) surrounding the outer needle across an exposed portion of the edge of the inner needle in a state before puncture where the edge of the inner needle protrudes from a tip of the outer needle,
wherein a sleeve (7) that abuts against the outer needle hub to prevent the outer needle hub from retracting axially rearward relative to the inner needle hub is provided outside the inner needle hub,
the protector is axially movably provided outside the sleeve, and
a first engagement mechanism (61-71) capable of elastically engaging the protector in an advanced end position axially forward relative to the sleeve and a second engagement mechanism (61-72) capable of engaging the protector in a retracted end position axially rearward relative to the sleeve are provided between the sleeve and the protector,
wherein an extending portion (50) extending farther outward from the protector is provided on the outer needle hub,
an inserting portion (51) into which the protector is axially inserted is formed in the extending portion,
a tip of the protector is placed farther forward from the edge of the inner needle protruding from the tip of the outer needle in said advanced end position of the protector,
the tip of the protector is placed in substantially the same position as the extending portion in said retracted end position of the protector, and
a retaining mechanism (52-62) capable of elastically preventing the protector from being withdrawn axially rearward from the inserting portion is provided between the outer needle hub and the protector,
wherein the inner needle hub is retractable axially rearward relative to the sleeve,
a stopper mechanism (72-32) for preventing further retraction of the inner needle hub relative to the sleeve when the inner needle hub is retracted relative to the sleeve to a predetermined position where the edge of the inner needle is inserted into the protector placed in said retracted end position is provided between the inner needle hub and the sleeve, and
when the inner needle hub is further retracted axially rearward from said predetermined position with said extending portion pressed, the protector is withdrawn axially rearward from the inserting portion against an engagement force by the retaining mechanism, by a pulling force axially rearward acting on the protector via the stopper mechanism, the sleeve, and the second engagement mechanism.

2. The indwelling needle according to claim 1, wherein a third engagement mechanism capable of elastically engaging the sleeve in the initial position where the outer needle hub is prevented from retracting axially rearward relative to the inner needle hub is provided between said sleeve and said inner needle hub.

3. The indwelling needle according to claim 2, wherein a first engagement hole at the tip and a second engagement hole at the bottom radially passing through are formed in said sleeve, a first engagement piece is formed at the bottom of said protector, which engages in the first engagement hole when the protector is placed in the advanced end position relative to the sleeve, and engages in the second engagement hole when the protector is placed in the retracted end position relative to the sleeve, and the first engagement piece and the first engagement hole constitute said first engagement mechanism, and the first engagement piece and the second engagement hole constitute said second engagement mechanism,
wherein a second engagement piece is formed at the tip of said inner needle hub, which engages in the first engagement hole when the sleeve is placed in said initial position, and engages in the second engagement hole when the inner needle hub is retracted to said predetermined position, the second engagement piece and the first engagement hole constitute said third engagement mechanism, and the second engagement piece and the second engagement hole constitute said stopper mechanism.

4. The indwelling needle according to claim 3, wherein an axial groove connecting the first engagement hole and the second engagement hole is formed in an outer periphey of said sleeve so as to guide said first engagement piece when said protector moves axially rearward from said advanced end position to said retracted end position relative to the sleeve, and
wherein an axial groove connecting the first engagement hole and the second engagement hole is formed in an inner periphery of the sleeve so as to guide said second engagement piece when the inner needle hub moves axially rearward from said initial position to said predetermined position relative to the sleeve.

5. The indwelling needle according to any one of claims 1 to 4, wherein a flange is formed at the bottom of said inner needle hub, and a substantially cylindrical grip extending axially forward and having an inner diameter larger than an outer diameter of said protector is formed on an outer periphery of the flange.

## Patentansprüche

1. Eine Verweilnadel (1) mit:
einer Innennadel (2) mit einer Kante (2a) an ihrem Vorderende,
eine Innennadelbuchse (3), die mit einem unteren Teil der Innennadel verbunden ist,
einer hohlen Außennadel (4), in welche die Innennadel verschiebbar eingesetzt ist,
einer Außennadelbuchse (5), die mit einem unteren Teil der Außennadel verbunden ist und
einem zylindrischen Protektor (6), der die Außennadel über einen freiliegenden Abschnitt der Kante der Innennadel in einem Zustand vor dem Einstich, bei dem die Kante der Innennadel von einem Vorderende der Außennadel vorsteht, umgibt,
wobei eine Hülse (7), welche an der Außennadelbuchse anliegt, um ein axiales Zurückziehen der Außennadelbuchse nach hinten relativ zu der Innennadelbuchse zu verhindern, außerhalb der Innennadelbuchse vorgesehen ist,
der Protektor axial beweglich außerhalb der Hülse vorgesehen ist, und
ein erster Eingriffsmechanismus (61-71), der mit dem Protektor in einer axial nach vorne relativ zu der Hülse vorgeschobenen Endposition elastisch in Eingriff gelangen kann, und ein zweiter Eingriffsmechanismus (61-72), der mit dem Protektor in einer axial nach hinten relativ zu der Hülse zurückgezogenen Endposition in Eingriff gelangen kann, zwischen der Hülse und dem Protektor vorgesehen sind,
wobei ein Erstreckungsabschnitt (50), der sich von dem Protektor weiter nach außen erstreckt, an der Außennadelbuchse vorgesehen ist,
ein Einsetzabschnitt (51), in welchen der Protektor axial eingesetzt ist, in dem Erstreckungsabschnitt ausgebildet ist,
ein Vorderende des Protektors weiter nach vorne gegenüber der Kante der von dem Vorderende der Außennadel in der vorgeschobenen Endposition des Protektors vorstehenden Innennadel angeordnet ist,
das Vorderende des Protektors in der im wesentlichen selben Position wie der Erstreckungsabschnitt in der zurückgezogenen Endposition des Protektors angeordnet ist, und
ein Rückhaltemechanismus (52-62), der ein axiales Zurückziehen des Protektors nach hinten von dem Einsetzabschnitt elastisch verhindern kann, zwischen der Außennadelbuchse und dem Protektor vorgesehen ist,
wobei die Innennadelbuchse axial nach hinten relativ zu der Hülse zurückziehbar ist,
ein Stoppermechanismus (72-32) zum Verhindern eines weiteren Zurückziehens der Innennadelbuchse relativ zu der Hülse, wenn die Innennadelbuchse relativ zu der Hülse in eine vorbestimmte Position zurückgezogen wird bzw. ist, an der die Kante der Innennadel in den in der zurückgezogenen Endposition befindlichen Protektor eingesetzt ist, zwischen der Innennadelbuchse und der Hülse vorgesehen ist, und
wenn die Innennadelbuchse von der vorbestimmten Position bei gedrücktem Erstreckungsabschnitt weiter axial nach hinten zurückgezogen wird, der Protektor durch eine axial nach hinten wirkende Zugkraft auf den Protektor über den Stoppermechanismus, die Hülse und den zweiten Eingriffsmechanismus von dem Einsetzabschnitt gegen eine Eingriffskraft durch den Rückhaltemechanismus axial nach hinten zurückgezogen wird.

2. Die Verweilnadel gemäß Anspruch 1, wobei ein dritter Eingriffsmechanismus, der mit der Hülse in der Ausgangsposition, an der die Außennadelbuchse an einem Zurückziehen axial nach hinten relativ zu der Innennadelbuchse gehindert ist, elastisch in Eingriff gelangen kann, zwischen der Hülse und der Innennadelbuchse vorgesehen ist.

3. Die Verweilnadel gemäß Anspruch 2, wobei ein erstes Eingriffsloch an dem Vorderende und ein zweites Eingriffsloch an dem unteren Teil radial hindurchverlaufend in der Hülse ausgebildet sind, ein erstes Eingriffsteil an dem unteren Teil des Protektors ausgebildet ist, welches in das erste Eingriffsloch eingreift, wenn der Protektor in der vorgeschobenen Endposition relativ zu der Hülse angeordnet ist, und in das zweite Eingriffsloch eingreift, wenn der Protektor in der zurückgezogenen Endposition relativ zu der Hülse angeordnet ist, und das erste Eingriffsteil und das erste Eingriffsloch den ersten Eingriffsmechanismus bilden, und das erste Eingriffsteil und das zweite Eingriffsloch den zweiten Eingriffsmechanismus bilden,
wobei ein zweites Eingriffsteil an dem Vorderende der Innennadelbuchse ausgebildet ist, welches in das erste Eingriffsloch eingreift, wenn die Hülse in der Ausgangsposition angeordnet ist, und in das zweite Eingriffsloch eingreift, wenn die Innennadelbuchse in die vorbestimmte Position zurückgezogen ist, wobei das zweite Eingriffsteil und das erste Eingriffsloch den dritten Eingriffsmechanismus bilden und das zweite Eingriffsteil und das zweite Eingriffsloch den Stoppermechanismus bilden.

4. Die Verweilnadel gemäß Anspruch 3, wobei eine axiale Nut, die das erste Eingriffsloch und das zweite Eingriffsloch verbindet, in einem Außenumfang der Hülse so ausgebildet ist, daß sie das erste Eingriffsteil führt, wenn der Protektor sich axial nach hinten aus der vorgeschobenen Endposition in die zurückgezogene Endposition relativ zu der Hülse bewegt, und
wobei eine axiale Nut, die das erste Eingriffsloch und das zweite Eingriffsloch verbindet, in einem Innenumfang der Hülse so ausgebildet ist, daß sie das zweite Eingriffsteil führt, wenn die Innennadelbuchse sich axial nach hinten von der Ausgangsposition in die vorbestimmte Position relativ zu der Hülse bewegt.

5. Die Verweilnadel gemäß einem der Ansprüche 1 bis 4,
wobei ein Flansch an dem unteren Teil der Innennadelbuchse ausgebildet ist, und ein im wesentlichen zylindrischer Griff, der sich axial nach vorne erstreckt und einen Innendurchmesser besitzt, der größer als ein Außendurchmesser des Protektors ist, an einem Außenumfang des Flansches ausgebildet ist.

## Revendications

1. Aiguille (1) à demeure comprenant :
une aiguille (2) intérieure ayant un bord (2a) à son extrémité ;
une tulipe (3) d'aiguille intérieure reliée à un fond de l'aiguille intérieure ;
une aiguille (4) extérieure creuse dans laquelle l'aiguille intérieure est insérée à coulissement ;
une tulipe (5) d'aiguille extérieure reliée à un fond de l'aiguille extérieure ; et
un protecteur (6) cylindrique entourant l'aiguille extérieure sur une partie dégagée du bord de l'aiguille intérieure dans un état avant piqûre où le bord de l'aiguille intérieure dépasse d'une pointe de l'aiguille extérieure,
dans laquelle un manchon (7), qui bute sur la tulipe d'aiguille extérieure pour empêcher la tulipe d'aiguille extérieure de se rétracter axialement vers l'arrière par rapport à la tulipe d'aiguille intérieure, est prévu à l'extérieur de la tulipe d'aiguille intérieure,
le protecteur est prévu mobile axialement à l'extérieur du manchon, et
un premier mécanisme (61 à 71) de coopération, apte à coopérer élastiquement avec le protecteur dans une position d'extrémité avancée axialement vers l'avant par rapport au manchon, et un deuxième mécanisme (61 à 72) de coopération, apte à coopérer avec le protecteur dans une position d'extrémité rétractée en arrière axialement par rapport au manchon, sont prévus entre le manchon et le protecteur,
dans laquelle une partie (50) d'extension s'étendant plus loin vers l'extérieur à partir du protecteur est prévue sur la tulipe d'aiguille extérieure,
une partie (51) d'insertion, dans laquelle le protecteur est inséré axialement, est formée dans la partie d'extension,
une pointe du protecteur est placée plus loin vers l'avant à partir du bord de l'aiguille intérieure dépassant de la pointe de l'aiguille extérieure dans la position d'extrémité avancée du protecteur,
la pointe du protecteur est placée sensiblement dans la même position que la partie d'extension en la position d'extrémité rétractée du protecteur, et
un mécanisme (52 à 62) de retenue, apte à empêcher élastiquement le protecteur d'être retiré axialement vers l'arrière à partir de la partie d'insertion, est prévu entre la tulipe d'aiguille extérieure et le protecteur,
dans laquelle la tulipe d'aiguille intérieure est rétractable axialement vers l'arrière par rapport au manchon,
un mécanisme (72 à 32) de butée pour empêcher un retrait supplémentaire de la tulipe d'aiguille intérieure par rapport au manchon, lorsque la tulipe d'aiguille intérieure est rétractée par rapport au manchon jusqu'à une position déterminée à l'avance où le bord de l'aiguille intérieure est insérée dans le protecteur placé dans la position d'extrémité rétractée, est prévu entre la tulipe d'aiguille intérieure et le manchon, et
lorsque la tulipe d'aiguille intérieure est rétractée davantage axialement vers l'arrière à partir de la position déterminée à l'avance, alors que la partie d'extension est comprimée, le protecteur est retiré axialement vers l'arrière à partir de la partie d'insertion à l'encontre d'une force de coopération par le mécanisme de retenue, par une force de traction agissant vers l'arrière axialement sur le protecteur par l'intermédiaire du mécanisme de butée, du manchon et du deuxième mécanisme de coopération.

2. Aiguille à demeure suivant la revendication 1,
dans laquelle un troisième mécanisme de coopération, apte à coopérer élastiquement avec le manchon dans la position initiale dans laquelle la tulipe de ligne extérieure est empêchée de se retirer axialement vers l'arrière par rapport à la tulipe d'aiguille intérieure, est prévu entre le manchon et la tulipe d'aiguille intérieure.

3. Aiguille à demeure suivant la revendication 2,
dans laquelle un premier trou de pénétration à la pointe et un deuxième trou de pénétration au fond traversant radialement sont formés dans le manchon, une première pièce de pénétration est formée au centre du protecteur, pièce qui pénètre dans le premier trou de pénétration, lorsque le protecteur est placé dans la position d'extrémité avancée par rapport au manchon, et qui pénètre dans le deuxième trou de pénétration, lorsque le protecteur est placé dans la position d'extrémité rétractée par rapport au manchon, et la première pièce de pénétration et le premier trou de pénétration constituent le premier mécanisme de coopération et la première pièce de pénétration et le deuxième trou de pénétration constitue le deuxième mécanisme de coopération,
dans laquelle une deuxième pièce de pénétration est formée à la pointe de la tulipe d'aiguille intérieure, pièce qui pénètre dans le premier trou de pénétration, lorsque le manchon est placé dans la position initiale, et qui pénètre dans le deuxième trou de pénétration, lorsque la tulipe d'aiguille intérieure est retirée jusqu'à la position déterminée à l'avance, la deuxième pièce de pénétration et le premier trou de pénétration constituant le troisième mécanisme de coopération et la deuxième pièce de pénétration et le deuxième trou de pénétration constituant le mécanisme de butée.

4. Aiguille à demeure suivant la revendication 3,
dans laquelle une gorge axiale, mettant le premier trou de pénétration et le deuxième trou de pénétration en communication, est formée dans une périphérie extérieure du manchon de manière à guider la première pièce de pénétration, lorsque le protecteur se déplace axialement vers l'arrière à partir de la position d'extrémité avancée pour aller à la position d'extrémité contractée par rapport au manchon, et
dans laquelle une gorge axiale, mettant le premier trou de pénétration et le deuxième trou de pénétration en communication, est formée dans une périphérie intérieure du manchon de manière à guider la deuxième pièce de pénétration, lorsque la tulipe d'aiguille intérieure se déplace axialement vers l'arrière de la position initiale à la position déterminée à l'avance par rapport au manchon.

5. Aiguille à demeure suivant l'une quelconque des revendications 1 à 4, dans laquelle une bride est formée au fond de la tulipe d'aiguille intérieure et une prise sensiblement cylindrique, s'étendant axialement vers l'avant et ayant un diamètre intérieur plus grand qu'un diamètre extérieur du protecteur, est formée sur une périphérie extérieure de la bride.
